# EUROPEAN PATENT APPLICATION

(11) **EP 4 442 223 A1**
(43) Date of publication of application: **09.10.2024**
(21) Application number: 22899963.7
(22) Date of filing: 15.08.2022
(51) Int. Cl.: A61F 2/14, G02B 7/00

(54) **ARTIFICIAL CORNEA BASED ON UMBRELLA-SHAPED CYLINDER, AND PREPARATION PROCESS THEREFOR**

(30) Priority: 02.12.2021 CN 202111463489
(71) Applicant: Beijing Microkpro Medical Instrument Co., Ltd., Beijing 102609 (CN)
(72) Inventor: YU, Ting, Beijing 102609 (CN)
(74) Representative: Ponti & Partners, S.L.P
(86) International application number: PCT/CN2022/112487
(87) International publication number: WO 2023/098143

(57) **Abstract**

An artificial cornea based on an umbrella-shaped cylinder (20), and a preparation process therefor. The artificial cornea comprises a support (10) and a cylinder (20) matchedly connected to the support (10); the support (10) comprises a main body structure (101) having a spherical surface structure; an opening (11) is provided in the middle of the main body structure (101); the cylinder (20) passes through the opening (11); micropores are formed on the peripheral surface of the main body structure (101); the top end face of the cylinder (20) is formed as a cambered umbrella-shaped structure; a concave spherical surface structure is formed on the bottom end face of the cylinder (20). According to the artificial cornea, by means of the micropores on the peripheral surface of the main body structure (101), healing tissue passes through the micropores, thereby increasing the binding force and stability between the support (10) and the cornea; second, by means of the cambered umbrella-shaped structure formed on the top end surface of the cylinder (20), during later use in a patient, continuously growing tissue can be prevented from climbing to the lens surface of the cylinder (20) to cover the lens surface, thus, light can enter the cylinder (20), the optical effect is normally exerted, and the occurrence of epiretinal membrane complications is reduced.

## Description

### TECHNICAL FIELD

The present invention relates a medical device, and specifically to a keratoprosthesis based on an umbrella-shaped optical cylinder and a preparation process therefor.

### BACKGROUND ART

The keratoprosthesis (artificial cornea) is a product which is made of medical polymer materials and is similar to a human cornea. The keratoprosthesis includes two parts, i.e., an optical cylinder (a cylinder) and a support. The optical cylinder, which is made of a transparent material with excellent optical properties and stable physical and chemical properties, is used to replace turbid cornea which obstructs an optical path of eyeball after lesion. The support is equivalent to a bridge connecting the optical cylinder and surrounding tissue, and thus is required to have good histocompatibility.

When a person's cornea cannot undergo living cornea transplantation due to damage, the keratoprosthesis needs to be installed. The keratoprosthesis is suitable for patients who have good fundus function but have corneal damage and cannot receive corneal transplantation, such as patients with cornea own disease, trauma or the like, and patients who become blind due to multiple times of failed corneal transplantation. At present, most of the keratoprosthesiss in the market are composed of a support made of a titanium plate or a titanium foil and a PMMA optical cylinder. However, in clinical practice, as the support of the keratoprosthesis is made of the titanium plate, the support has poor binding and stability between human corneal lamellae and is easy to fall off; in addition, the optical cylinder of the keratoprosthesis is of a cylindrical structure, and a lens surface, in later use by the patients, is easy to be closed up by recovery tissue of wound, and the lens surface is covered, so that light cannot enter the optical cylinder, thus optical effect cannot be exerted.

### SUMMARY

The present invention aims at providing an keratoprosthesis based on an umbrella-shaped optical cylinder and a preparation process therefor. The keratoprosthesis based on an umbrella-shaped optical cylinder can normally exert optical effect and reduce occurrence of epiretinal membrane complications.

In order to achieve the above objective, technical solutions used in the present invention are as follows. Provided is an keratoprosthesis based on an umbrella-shaped optical cylinder, including a support and an optical cylinder fitted and connected with the support, wherein the support includes a main body structure of a spherical structure, an opening is formed in the middle of the main body structure, and the optical cylinder is provided in the opening, wherein micropores are formed on a circumferential surface of the main body structure, a top end surface of the optical cylinder is formed into a cambered umbrella-shaped structure, a bottom end surface of the optical cylinder is formed into a concave spherical structure, and after the support is fitted and mounted with the optical cylinder, a protruding direction of the cambered umbrella-shaped structure of the top end surface of the optical cylinder is consistent with a protruding direction of the spherical structure of the main body structure.

As a further improvement, the optical cylinder is made of a PMMA material, and the optical cylinder includes a light guiding section, wherein a top end surface of the light guiding section horizontally extends outwards to form an extension surface, a top end surface of the light guiding section has a bulge in the middle, and the bulge is formed into the camber-shaped structure along an outer end portion of the extension surface.

As a further improvement, the optical cylinder is of a stepped cylindrical structure, and the optical cylinder includes an insertion section, a mounting section formed on the insertion section, and the light guiding section formed on the mounting section, wherein a bottom end surface of the insertion section is formed into the concave spherical structure.

As a further improvement, a guiding surface is formed at a joint between the insertion section and the mounting section, and an outer diameter of the mounting section is equivalent to an inner diameter of the opening.

As a further improvement, the main body structure is made of a PMMA material, and the main body structure is formed with the micropores.

As a further improvement, the main body structure is made of a microporous titanium plate or a titanium mesh material, and the micropores are formed on the circumferential surface of the main body structure.

As a further improvement, an outer peripheral dimension of the support is 7-8.5 mm, and a thickness of the support is 0.05-0.5 mm.

As a further improvement, an internal thread is formed at an inner circumferential wall of the opening, and an outer circumferential surface of the mounting section of the optical cylinder is provided with an external thread engaged with the internal thread of the opening.

The present invention further discloses a preparation process for keratoprosthesis based on an umbrella-shaped optical cylinder, specifically including steps of:
S1: preparing an optical cylinder, wherein by lathe turning, the optical cylinder made of a PMMA material is formed into a stepped cylindrical structure, a top end surface of the optical cylinder is of an umbrella-shaped structure and a bottom end surface of the optical cylinder is formed into a concave spherical structure, and a mounting section is turned to form a mounting external thread;
S2: preparing a main body structure in a support, wherein a PMMA electrospinning plate is prepared by PMMA electrospinning and 3D printing technology, to realize formation of the main body structure in the support, and then soluble small molecules therein are dissolved by a physical mixing method, so as to realize micropores on the support;
S3: performing stamping on plate, to form the main body structure into a spherical structure with an opening provided in the middle;
S4: performing lathe turning, to turn an inner circumferential wall of the opening of the main body structure to form an internal thread engaged with the external thread of the mounting section; and
S5: fitting the support and the optical cylinder, wherein the optical cylinder is fitted and mounted into the opening of the main body structure through the external thread on the mounting section of the optical cylinder.

The present invention further discloses a preparation process for keratoprosthesis based on an umbrella-shaped optical cylinder according to another embodiment, specifically including steps of:
S1: preparing an optical cylinder, wherein by lathe turning, the optical cylinder made of a PMMA material is formed into a stepped cylindrical structure, a top end surface of the optical cylinder is of an umbrella-shaped structure and a bottom end surface of the optical cylinder is formed into a concave spherical structure, and a mounting section is turned to form a mounting external thread;
S2: preparing a main body structure in a support, wherein a microporous titanium plate is prepared by adding an inorganic substance such as hydroxyapatite, and after formation of the main body structure, the inorganic substance is dissolved by a solvent, so as to render the titanium plate or titanium mesh of a microporous structure;
S3: performing stamping on plate, to form the main body structure into a spherical structure with an opening provided in the middle;
S4: performing lathe turning, to turn an inner circumferential wall of the opening of the main body structure to form an internal thread engaged with the external thread of the mounting section; and
S5: fitting the support and the optical cylinder, wherein the optical cylinder is fitted and mounted into the opening of the main body structure through the external thread on the mounting section of the optical cylinder.

The above technical solutions of the present invention have the following beneficial effects: for the keratoprosthesis based on an umbrella-shaped optical cylinder in the present invention, through the micropores on the circumferential surface of the main body structure of the support, healing tissue is allowed to pass through the micropores, thus increasing a binding force and stability between the support and the cornea; secondly, through the cambered umbrella-shaped structure formed by the top end surface of the optical cylinder, during later use by the patients, continuously growing tissue is prevented from climbing onto the lens surface of the optical cylinder to cover the lens surface, so that it is easy for light to enter the optical cylinder, the optical effect is normally exerted, and the occurrence of epiretinal membrane complications is reduced.

As a further improvement, the support of the keratoprosthesis based on an umbrella-shaped optical cylinder further includes clamping structures formed on a periphery of the main body structure.

As a further improvement, the clamping structures are integrally formed on the main body structure, and are formed on the periphery of the main body structure in an annular array with a center of sphere of the main body structure as a center.

As a further improvement, the clamping structures are clover-shaped or double-wing-shaped structures.

As a further improvement, a plurality of through holes are uniformly provided on the circumferential surface of the main body structure, and the plurality of through holes are provided on the circumferential surface of the main body structure in an annular array with a hole center of the opening as a center, and wherein an aperture of each of the through holes is 0.5 mm.

### BRIEF DESCRIPTION OF DRAWINGS

FIG. 1 is a structural schematic view of keratoprosthesis based on an umbrella-shaped optical cylinder in the present invention;
FIG. 2 is a front view of a support 10 in a first embodiment of the present invention;
FIG. 3 is a front view of a main body structure 101 of the support 10 in FIG. 2;
FIG. 4 is a top view of FIG. 3;
FIG. 5 is a front view of the support 10 in a second embodiment of the present invention;
FIG. 6 is a front view of the main body structure 101 of the support 10 in FIG. 5; and
FIG. 7 is a top view of FIG. 6.

### DETAILED DESCRIPTION OF EMBODIMENTS

Embodiments of the present invention are illustrated in detail below with reference to the drawings and examples.

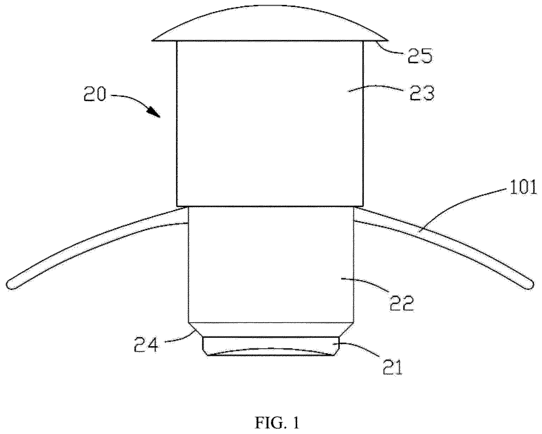

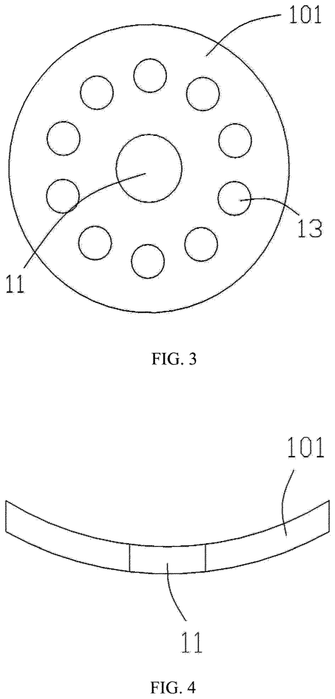

Referring to FIG. 1 to FIG. 4, keratoprosthesis based on an umbrella-shaped optical cylinder in a first embodiment of the present invention includes a support 10 and an optical cylinder 20 fitted and connected with the support 10 for corneal transplantation.

The support 10 is configured for bearing and mounting the optical cylinder 20, and the support 10 includes a main body structure 101 and clamping structures 102 formed on a periphery of the main body structure 101.

The main body structure 101 is of a spherical structure, an opening 11 is formed in the middle of the main body structure 101, and the optical cylinder 20 is provided in and passes through the opening 11. Specifically, an internal thread is formed on an inner circumferential wall of the opening 11 for fitting and mounting the optical cylinder 20. In the present embodiment, the support 10 has an outer peripheral dimension of 7-8.5 mm and a thickness of 0.05-0.5 mm.

Micropores are formed on a circumferential surface of the main body structure 101. With the micropores on the circumferential surface of the main body structure 101, it is easy for healing tissue to pass through the micropores, thus increasing a binding force and stability between the support and the cornea, and preventing falling off. In the present embodiment, a plurality of through holes 13 are uniformly formed on the circumferential surface of the main body structure 101. Preferably, ten through holes 13 are provided, an aperture of each through hole 13 is 0.5 mm, and the plurality of through holes 13 are formed on the circumferential surface of the main body structure 101 in an annular array with a hole center of the opening 11 as a center.

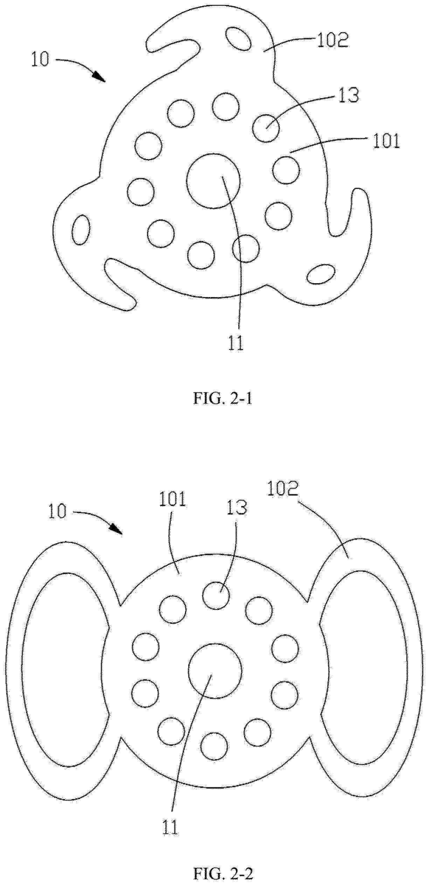

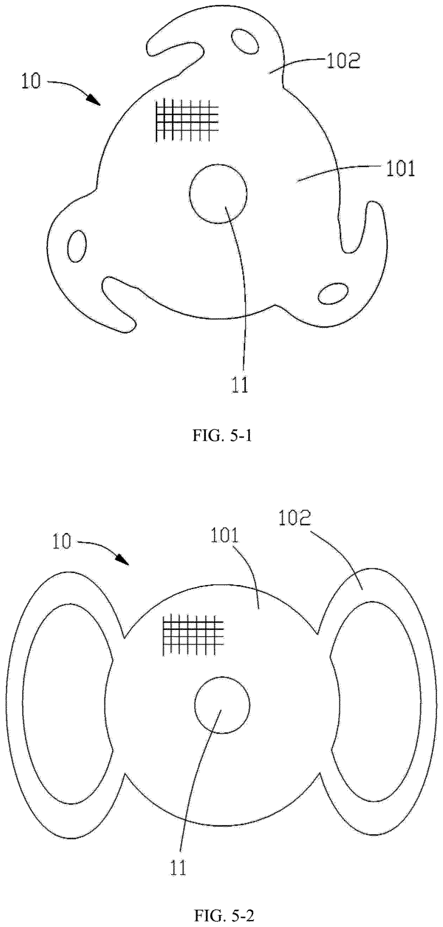

The clamping structures 102 are integrally formed on the main body structure 101, and are formed on the periphery of the main body structure 101 in an annular array with a center of sphere of the main body structure 101 as a center. In the present embodiment, the clamping structures 102 are structures in a clover shape, with reference to FIG. 2-1 or FIG. 5-1, or in a double-wing shape, with reference to FIG. 2-2 or FIG. 5-2, etc.

The support 10 is made of a PMMA material, and the main body structure 101 is processed by PMMA electrospinning and 3D printing technology to realize formation of the support, and then soluble small molecules therein are dissolved by a physical mixing method, to realize a microporous structure on the support.

The optical cylinder 20 is mounted in the opening 11 of the main body structure 101, so that during later use by the patients, continuously growing tissue is prevented from climbing onto the lens surface of the optical cylinder to cover the lens surface, thus it is easy for light to enter the optical cylinder, the optical effect is normally exerted, and the occurrence of epiretinal membrane complications is reduced. Specifically, a top end surface of the optical cylinder 20 has a bulge in the middle, the bulge forms a cambered umbrella-shaped structure along an outer end portion of the top end surface, and the optical cylinder 20 is of a solid structure in the interior. In the present embodiment, the top end surface of the optical cylinder 20 is of the cambered umbrella-shaped structure, curvature of an arc line of a top end of a longitudinal section of the optical cylinder 20 is 7.6 mm, and a chord length corresponding to the arc line is 3-6 mm, preferably 4.5 mm. Through the cambered umbrella-shaped structure formed by the top end surface of the optical cylinder 20, it facilitates fixing and holding of the support during surgery, and during later use by the patients, the continuously growing tissue can be prevented from climbing onto the lens surface of the optical cylinder to cover the lens surface, so that it is easy for light to enter the optical cylinder, the optical effect is normally exerted, and the occurrence of epiretinal membrane complications is reduced.

The optical cylinder 20 is of a stepped cylindrical structure made of a PMMA material. The optical cylinder 20 includes an insertion section 21, a mounting section 22 formed on the insertion section 21 and a light guiding section 23 formed on the mounting section 22. The insertion section 21, the mounting section 22 and the light guiding section 23 are coaxial and have successively increased radial dimensions. A guiding surface 24 is formed at a joint between the insertion section 21 and the mounting section 22. An outer diameter of the mounting section 22 is equivalent to an inner diameter of the opening 11, and an outer circumferential surface of the mounting section 22 is provided with an external thread engaged with the internal thread of the opening 11, so that the optical cylinder 20 is mounted in the opening 11.

A bottom end surface of the insertion section 21 is formed into a concave spherical structure. A top end surface of the light guiding section 23 is formed into a convex umbrella-shaped structure. Specifically, the top end surface of the light guiding section 23 horizontally extends outwards to form an extension surface 25, and a bulge of the top end surface of the light guiding section 23 forms a camber-shaped structure along an outer end portion of the extension surface 25. Moreover, after the support 10 is fitted and mounted with the optical cylinder 20, a protruding direction of the cambered umbrella-shaped structure of the top end surface of the optical cylinder 20 is consistent with a protruding direction of the spherical structure of the main body structure 101 of the support 10.

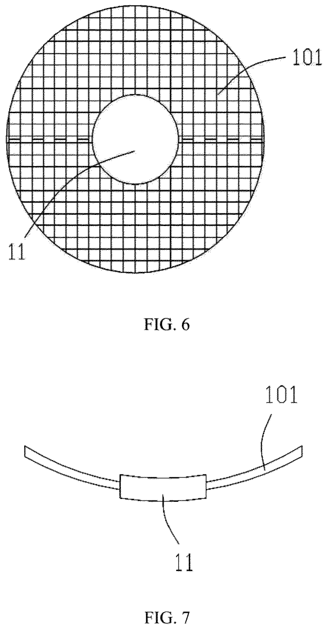

Referring to FIG. 1 and FIG. 5 to FIG. 7 in combination, keratoprosthesis based on an umbrella-shaped optical cylinder in a second embodiment of the present invention is substantially the same as that in the first embodiment in structure, and a difference lies in that the main body structure 101 of the support 10 is made of a microporous titanium plate or a titanium mesh material.

The main body structure 101 is of a spherical structure, and micropores are formed on the main body structure 101, so that the healing tissue passes through the micropores, thus increasing the binding force between the support and the cornea and preventing falling off. An opening 11 is provided in the middle of the main body structure 101, and an optical cylinder 20 is provided in and passes through the opening 11. The support 10 has an outer peripheral dimension of 8 mm and a thickness of 0.1 mm.

During formation of the titanium plate or the titanium mesh of the main body structure 101, an inorganic substance such as hydroxyapatite is added, and after the formation, the inorganic substance is dissolved by a solvent to render the titanium plate or the titanium mesh of a microporous structure. With use of the microporous structure formed on the main body structure 101, where an aperture of each single pore of the microporous structure is 3 nm-100 µm, the healing tissue is allowed to pass through the micropores, thus increasing a binding force and stability between the support and the cornea, and prevent falling off.

A preparation process for keratoprosthesis based on an umbrella-shaped optical cylinder includes following steps:
Step 1: preparation of an optical cylinder 20, which specifically is lathe turning, wherein a rotating speed of a turning tool is 2000-10000 r/min, and in the present embodiment, the rotating speed of the turning tool is preferably 8000 r/min, to implement processing of an optical lens surface and a micro-fine structure, so that the optical cylinder 20 is formed into a stepped cylindrical structure, a top end surface of the optical cylinder 20 is of an umbrella-shaped structure, and a bottom end surface of the optical cylinder 20 is formed into a concave spherical structure, and a mounting section 22 is turned to form a mounting external thread.
Step 2: preparation of a main body structure 101 in a support 10, specifically including preparation of a microporous titanium plate or a PMMA electrospinning plate. The microporous titanium plate is prepared by adding an inorganic substance such as hydroxyapatite, and after formation, the inorganic substance is dissolved by a solvent, so as to render the titanium plate or the titanium mesh of a microporous structure. The PMMA electrospinning plate is prepared by PMMA electrospinning and 3D printing technology, to realize formation of the support, and then soluble small molecules therein are dissolved by a physical mixing method, so as to realize micropores on the support.
Step 3: performing stamping on plate, so that the main body structure 101 is formed into a spherical structure and is formed with an opening 11 in the middle.
Step 4: lathe turning, so that an inner circumferential wall of the opening 11 in the middle of the main body structure 101 is turned to form an internal thread engaged with the external thread of the mounting section 22.
Step 5: fitting and mounting the optical cylinder 20 into the opening 11 of the main body structure 101 through the external thread on the mounting section 22 of the optical cylinder 20.

In conclusion, for the keratoprosthesis based on an umbrella-shaped optical cylinder in the present invention, through the micropores on the circumferential surface of the main body structure 101 of the support 10, the healing tissue is allowed to pass through the micropores, thus increasing the binding force between the support and the cornea; secondly, through the umbrella-shaped structure formed by the top end surface of the optical cylinder 20, it facilitates fixing and holding of the support during surgery, and during later use by the patients, the continuously growing tissue can be prevented from climbing onto the lens surface of the optical cylinder to cover the lens surface, so that it is easy for light to enter the optical cylinder, the optical effect is normally exerted, and the occurrence of epiretinal membrane complications is reduced.

The embodiments described above are merely preferred embodiments of the present invention but not to limit the scope of the present invention. For those ordinarily skilled in the art, various alterations and improvements made to the technical solutions of the present invention, without departing from the design spirit of the present invention, should fall within the scope of protection defined in the claims of the present invention.

## Claims

1. A keratoprosthesis based on an umbrella-shaped optical cylinder, **characterized by** comprising a support (10) and an optical cylinder (20) fitted and connected with the support (10), wherein the support (10) comprises a main body structure (101) of a spherical structure, an opening (11) is formed in the middle of the main body structure (101), and the optical cylinder (20) is provided in the opening (11), wherein micropores are formed on a circumferential surface of the main body structure (101), a top end surface of the optical cylinder (20) is formed into a cambered umbrella-shaped structure, a bottom end surface of the optical cylinder (20) is formed into a concave spherical structure, and after the support (10) is fitted and mounted with the optical cylinder (20), a protruding direction of the cambered umbrella-shaped structure of the top end surface of the optical cylinder (20) is consistent with a protruding direction of the spherical structure of the main body structure (101).

2. The keratoprosthesis based on an umbrella-shaped optical cylinder according to claim 1, **characterized in that** the optical cylinder (20) is made of a PMMA material, and the optical cylinder (20) comprises a light guiding section (23), wherein a top end surface of the light guiding section (23) horizontally extends outwards to form an extension surface (25), a top end surface of the light guiding section (23) has a bulge in the middle, and the bulge is formed into the camber-shaped structure along an outer end portion of the extension surface (25).

3. The keratoprosthesis based on an umbrella-shaped optical cylinder according to claim 2, **characterized in that** the optical cylinder (20) is of a stepped cylindrical structure, and the optical cylinder (20) comprises an insertion section (21), a mounting section (22) formed on the insertion section (21), and the light guiding section (23) formed on the mounting section (22), wherein a bottom end surface of the insertion section (21) is formed into the concave spherical structure.

4. The keratoprosthesis based on an umbrella-shaped optical cylinder according to claim 3, **characterized in that** a guiding surface (24) is formed at a joint between the insertion section (21) and the mounting section (22), and an outer diameter of the mounting section (22) is equivalent to an inner diameter of the opening (11).

5. The keratoprosthesis based on an umbrella-shaped optical cylinder according to claim 1, **characterized in that** the main body structure (101) is made of a PMMA material, and the main body structure (101) is formed with the micropores.

6. The keratoprosthesis based on an umbrella-shaped optical cylinder according to claim 1, **characterized in that** the main body structure (101) is made of a microporous titanium plate or a titanium mesh material, and the micropores are formed on the circumferential surface of the main body structure (101).

7. The keratoprosthesis based on an umbrella-shaped optical cylinder according to claim 1, **characterized in that** an outer peripheral dimension of the support (10) is 7-8.5 mm, and a thickness of the support (10) is 0.05-0.5 mm.

8. The keratoprosthesis based on an umbrella-shaped optical cylinder according to claim 5, **characterized in that** an internal thread is formed at an inner circumferential wall of the opening (11), and an outer circumferential surface of the mounting section (22) of the optical cylinder (20) is provided with an external thread engaged with the internal thread of the opening (11).

9. A preparation process for a keratoprosthesis based on an umbrella-shaped optical cylinder, for producing the keratoprosthesis based on an umbrella-shaped optical cylinder according to any one of claims 1-5, 7 and 8, **characterized by** comprising steps of:
S 1: preparing the optical cylinder (20), wherein by lathe turning, the optical cylinder (20) made of a PMMA material is formed into a stepped cylindrical structure, the top end surface of the optical cylinder (20) is of an umbrella-shaped structure and the bottom end surface of the optical cylinder (20) is formed into the concave spherical structure, and a mounting section (22) is turned to form a mounting external thread;
S2: preparing the main body structure (101) in the support (10), wherein a PMMA electrospinning plate is prepared by PMMA electrospinning and 3D printing technology, to realize formation of the main body structure in the support, and then soluble small molecules therein are dissolved by a physical mixing method, so as to realize the micropores on the support;
S3: performing stamping on plate, to form the main body structure (101) into the spherical structure with the opening (11) provided in the middle;
S4: performing lathe turning, to turn an inner circumferential wall of the opening (11) of the main body structure (101) to form an internal thread engaged with the external thread of the mounting section (22); and
S5: fitting the support and the optical cylinder, wherein the optical cylinder (20) is fitted and mounted into the opening (11) of the main body structure (101) through the external thread on the mounting section (22) of the optical cylinder (20).

10. A preparation process for a keratoprosthesis based on an umbrella-shaped optical cylinder, for producing the keratoprosthesis based on an umbrella-shaped optical cylinder according to any one of claims 1-4 and 6-8, **characterized by** comprising steps of:
S 1: preparing an optical cylinder (20), wherein by lathe turning, the optical cylinder (20) made of a PMMA material is formed into a stepped cylindrical structure, a top end surface of the optical cylinder (20) is of an umbrella-shaped structure and a bottom end surface of the optical cylinder (20) is formed into a concave spherical structure, and a mounting section (22) is turned to form a mounting external thread;
S2: preparing the main body structure (101) in the support 10, wherein a microporous titanium plate is prepared by adding an inorganic substance such as hydroxyapatite, and after formation of the main body structure, the inorganic substance is dissolved by a solvent, so as to render the titanium plate or titanium mesh of a microporous structure;
S3: performing stamping on plate, to form the main body structure (101) into the spherical structure with the opening (11) provided in the middle;
S4: performing lathe turning, to turn an inner circumferential wall of the opening (11) of the main body structure (101) to form an internal thread engaged with the external thread of the mounting section (22); and
S5: fitting the support and the optical cylinder, wherein the optical cylinder (20) is fitted and mounted into the opening (11) of the main body structure (101) through the external thread on the mounting section (22) of the optical cylinder (20).

11. The keratoprosthesis based on an umbrella-shaped optical cylinder according to claim 1, **characterized in that** the support (10) comprises clamping structures (102) formed on a periphery of the main body structure (101).

12. The keratoprosthesis based on an umbrella-shaped optical cylinder according to claim 11, **characterized in that** the clamping structures (102) are integrally formed on the main body structure (101), and are formed on the periphery of the main body structure (101) in an annular array with a center of sphere of the main body structure (101) as a center.

13. The keratoprosthesis based on an umbrella-shaped optical cylinder according to claim 12, **characterized in that** the clamping structures (102) are of a clover-shaped or a double-wing-shaped structure.

14. The keratoprosthesis based on an umbrella-shaped optical cylinder according to claim 5, **characterized in that** a plurality of through holes (13) are uniformly formed on the circumferential surface of the main body structure (101), and the plurality of through holes (13) are formed on the circumferential surface of the main body structure (101) in an annular array with a hole center of the opening (11) as a center, wherein an aperture of each of the through holes (13) is 0.5 mm.
